# EUROPEAN PATENT APPLICATION

(11) **EP 0 926 157 A1**
(43) Date of publication of application: **30.06.1999**
(21) Application number: 98929678.5
(22) Date of filing: 25.06.1998
(51) Int. Cl.: C07K 14/47, C12N 9/14, C12N 15/55, C12Q 1/68, C07K 16/40

(54) **PROTEIN CAPABLE OF COMBINING WITH TBP TO FORM COMPLEX, POLYNUCLEOTIDE CODING FOR SAID PROTEIN, ANTI-SENSE POLYNUCLEOTIDE OF SAID POLYNUCLEOTIDE, AND ANTIBODY CAPABLE OF RECOGNIZING SAID PROTEIN**

(30) Priority: 27.06.1997 JP 18739897
(71) Applicant: Sumitomo Electric Industries, Ltd., Osaka-shi, Osaka 541-0041 (JP)
(72) Inventor: KISHIMOTO, Toshihiko; Yokohama Works of Sumitomo, Yokohama-shi; Kanagawa 244-8588 (JP); NIWA, Shin-ichiro; Yokohama Works of Sumitomo, Yokohama-shi; Kanagawa 244-8588 (JP); TAMURA, Taka-aki Chiba University, Faculty of, Chiba-shi Chiba 263-0022 (JP); MAKINO, Yasutaka Chiba University, Faculty of, Chiba-shi Chiba 263-0022 (JP)
(74) Representative: Baldock, Sharon Claire
(86) International application number: JP9802836
(87) International publication number: WO9900419

(57) **Abstract**

The present invention encompasses proteins comprising amino acid sequences set forth in SEQ ID NO: 3 in Sequence Listing, mutants thereof, and their homologue proteins. The proteins of the present invention have not only an ability to form a complex with TBP but also ATPase activity and DNA helicase activity. The present invention also encompasses genes coding the above-mentioned proteins. The present invention further encompasses antibodies specifically combining the above-mentioned proteins.

## Description

### TECHNICAL FIELD

This invention relates to a protein existing commonly in mammal tissues and forming a complex with TBP, polynucleptid coding the protein, antisense polynucleotide corresponding to the polynucleotide and antibody recognizing the protein.

### BACKGROUND ART

Carcinoma or cancer is known to occur because of some abnormality in a gene. A change or abnormality in the gene at its transcription level, in particular, is regarded as a major cause of cancer. Investigating a gene which is responsible for tumorogenesis is an indispensable important subject to elucidate the mechanism of onset of carcinoma or realize development of diagnosis method of cancer or treatment of cancer. Seeing such a gene has been performed eagerly since about 1980.

On the other hand, as a major factor of gene transcription, TBP (TATA binding protein) has been known to be involved in the transcription of any genes in organisms. Specifically, it has been reported that, when DNA is transcribed to mRNA, TBP binds to a promoter region of the DNA, thereby controlling the transcription of genes. Then, TBP is assumed to have functions corresponding to different transcription controls of individual genes. It is presumed that TBP forms complexes with other various proteins, thereby yielding the individual functions depending on the configurations of their associating proteins or complexes.

A wide range of the above-mentioned proteins forming complexes with TBP have conventionally been identified, such as TAFs (TBP associating factors), Dr1, and Dr2, which are considered to relate to gene transcription, as well as cancer-related genes such ad Tat, p53, and Rb, and so forth.

On the other hand, among the inventors, Tamura and Makino have elucidated a method of acquiring proteins expressed in only a minute amount or proteins having a low antigenicity and genes coding them ("Bio-Manual Series 5, Transcription Factor Studying Method," pp. 11 to 16, pp. 27-37, pp. 189-196, and pp. 215-219 (Yodosha, 1993).

By using the above-mentioned method, the inventors have disclosed in Japanese Patent Application Laid-Open No. 9-77792, as a protein which binds to TBP and specifically increases its expression in cancer, TIP120 protein, a gene coding this protein, and an antibody for this protein.

### DISCLOSURE OF THE INVENTION

An object of this invention is to acquire a novel protein forming a complex with TBP which is a major factor of gene transcription.

Also, another object of this invention is to provide a gene coding said protein and to provide an antibody recognizing said protein.

Further, another object of this invention is to realize detecting said protein using said antibody.

Further, another object of this invention is to provide a method to acquire a homologue of said protein forming a complex with TBP, a homologous protein acquired by said method and a gene coding said homologous protein.

By using a method improving the above-mentioned method of Tamura and Makino, the inventors have determined the base sequence of a gene (TIP49 gene) for a protein which forms a complex with TBP and has a molecular weight of 49 KD (the protein being named TIP49). Also, the inventors have determined an amino acid sequence coded by this gene.

Further, a recombinant protein coded by TIP49 gene has been prepared, and it has been confirmed that this recombinant protein has the same characteristics as TIP49 which exists in nature.

Also, using DNA which is a part of TIP49 genes as a probe, from a cDNA library which is different from the cDNA library from which the probe DNA has been acquired, cDNA (homologue of TIP49 gene) to which the probe hybridized has been acquired by the probe has been acquired, and the amino acid sequence coded by this cDNA has been determined.

Also, by immunizing with TIP49 protein an animal other than the animal from which this protein is originated, an antibody recognizing this protein has been acquired, and the antigenicity of the protein has been confirmed.

This invention provides the following:
1. A protein selected from the following groups (a) and (b):
   (a) a protein comprising the amino acid sequence set forth in SEQ ID NO.1 in the Sequence Listing;
   (b) a protein having the activity of forming a complex with TBP and comprising the amino acid sequence modified by substitution, deletion or addition of one or plural amino acid(s) in the amino acid sequence set forth in SEQ ID NO.1 in the Sequence Listing.
2. A protein selected from the following groups (c) and (d):
   (a) a protein comprising the amino acid sequence set forth in SEQ ID NO.3 in the Sequence Listing;
   (b) a protein having the activity of forming a complex with TBP and comprising the amino acid sequence modified by substitution, deletion or addition of one or plural amino acid(s) in the amino acid sequence set forth in SEQ ID NO.3 in the Sequence Listing.
3. A protein of the aforementioned item 1 or 2 further having the ATPase activity.
4. A protein of any one of the aforementioned items 1 to 3 further having DNA helicase activity.
5. A protein selected from the following groups (e) and (f):
   (e) a protein having the activity of forming a complex with TBP and comprising a part of the amino acid sequence set forth in SEQ ID NO.1 in the Sequence Listing;
   (f) a protein having the activity of forming a complex with TBP and comprising a part of the amino acid sequence modified by substitution, deletion or addition of one or plural amino acid(s) in the amino acid sequence set forth in SEQ ID NO.1 in the Sequence Listing.
6. A protein selected from the following groups (g) and (h):
   (g) a protein having the activity of forming a complex with TBP and comprising a part of the amino acid sequence set forth in SEQ ID NO.3 in the Sequence Listing;
   (h) a protein having the activity of forming a complex with TBP and comprising a part of the amino acid sequence modified by substitution, deletion or addition of one or plural amino acid(s) in the amino acid sequence set forth in SEQ ID NO.3 in the Sequence Listing.
7. A protein of the aforementioned item 5 or 6 further having the ATPase activity.
   Further this invention provides the following polynucleotides.
8. A protein of any one of the aforementioned items 5 to 7 further having DNA helicase activity.
9. DNA coding the protein of any one of the aforementioned items 1 to 8.
10. DNA comprising the base sequence set forth in SEQ ID NO.2 or 4.
11. RNA coded by the DNA of the aforementioned item 9 or 10.
12. An antisense DNA corresponding to the DNA of the item 9 or 10, an antisense RNA corresponding to the RNA of the aforementioned item 11, or an antisense polynucleotide comprising a derivative thereof.
13. A polynucleotide comprising a part of the polynucleotide of any one of the aforementioned items 9 to 12 and comprising 12 or more consecutive bases.
14. A polynucleotide comprising a part of the polynucleotide of any one of the aforementioned items 9 to 12 and comprising 16 or more consecutive bases.
15. A polynucleotide which is any one of an antisense DNA corresponding to the DNA coding the protein of any one of the aforementioned items 5 or 8, an antisense RNA corresponding to the RNA coding the protein of any one of the aforementioned items 5 or 8, or an antisense polynucleotide comprising a derivative thereof, wherein the polynucleotide has GC content of 30 to 70%.
16. A polynucleotide which is the polynucleotide of any one of the aforementioned items 9 to 15 as chemically modified.
17.A method for obtaining cDNA which is a homologue of the DNA of the aforementioned item 10, wherein using the polynucleotide of the aforementioned item 13 or 15 as a probe, cDNA hybridizing to said polynucleotide is obtained from a cDNA library.
18. cDNA which is a homologue of the DNA comprising the base sequence set forth in SEQ ID NO.2 or 4 and which is obtained by said method obtaining cDNA.
19. A protein which is a homologue of the protein comprising the amino acid sequence set forth in SEQ ID NO.1 or 3, wherein the protein comprises an amino acid sequence coded by said cDNA.
20. An antibody (embracing polyclonal antibody and monoclonal antibody) recognizing the protein of any one of the aforementioned items 1 to 8 and 19.
21. An active fragment of monoclonal antibody recognizing the protein of any one of the aforementioned items 1 to 8 and 19.
22. A method for detecting the protein of any one of the aforementioned items 1 to 8 and 19, characterized in using the antibody recognizing said proteins.

### BRIEF DESCRIPTION OF THE DRAWINGS

Fig.1 is a photograph showing the results of two dimensional gel electrophoresis of rat hepatic cell extract liquid. M represents a molecular weight marker. An arrow indicates the dot the amino acid sequence of which was determined.
Fig.2 is a graph showing the amino acid sequence of rat TIP49 protein and the base sequence of rat TIP49 gene.
Fig.3 is a graph showing homology between rat TIP49 protein and protozoan RuvB protein, and homology between rat TIP49 gene and protozoan RuvB gene respectively.
Fig.4 is a photograph showing the results of Northern blot hybridization using gel electrophoresis of polyA RNA originated from rat hepatic cell by use of a part of TIP49 gene as a probe.
Fig.5 is a photograph showing the results of Northern blot hybridization using gel electrophoresis of polyA RNA originated from various tissues of rat by use of a part of TIP49 gene as a probe.
Fig.6 is a view showing pET3a-His vector into which histidine tag was transduced and which was used in Example 4 to transduce TIP49 gene.
Fig.7 is a photograph showing the results of gel electrophoresis of the cell extract and purified protein thereof which were obtained from E.coli in which recombinant TIP49 was expressed.
Fig.8A is a chart of the column pattern of MonoQ by which TIP49 protein was purified.
Fig.8B is a photograph showing the results of gel electrophoresis of purified TIP49 protein(stained by Coomassie Brilliant Blue).
Fig.9 is a photograph showing the results of analysis of rat hepatic cell nuclear extract liquid, crude TIP49 from said nuclear extract liquid and recombinant TIP49 purified by N-NTA agarose column, by Western blotting method using anti TIP49 antibody.
Fig.10 is a photograph showing the results of analysis by Western blotting of precipitate, supernatant and rat hepatic cell extract; said precipitate and said supernatant were obtained by using anti HA antibody immunoprecipitating nuclear extract liquid obtained by expressing HA-TBP in animal cell. In said analysis as first antibody, anti TBP antibody, anti HA antibody or anti TIP49 antibody was used in the case of the upper column A of Fig.10, the middle column B or the bottom column C respectively.
Fig.11 is a graph showing the results of measurement, by a liquid scintillation counter, of ATPase activity of TIP49 purified by MonoQ.
Fig.12 is a graph showing the results of measurement of ATPase activity of TIP49 purified by MonoQ, measured by a liquid scintillation counter under the conditions in which various nucleic acids were added to the solution of TIP49.
Fig.13 is a photograph showing the results of gel electrophoresis of the reactant obtained by reaction between a sample DNA and TIP49 purified by MonoQ.

### Best Modes for Carrying Out the Invention

In the present invention, as will be explained later in Examples, TIP49 forming a complex with TBP has been isolated from a rat liver, and human TIP49 gene has further been acquired as shown in Example 10. Since TBP is a major factor for the transcription of any genes of organisms, it is presumed, with a very high possibility, that organisms other than rats and humans may have a protein having the same functions as those of the currently isolated protein. Therefore, by the method disclosed in Example 10, homologues of TIP49 are obtained from organisms other than humans or rats. Also, TIP49 gene widely exists in various tissues as verified in Example 4, and TIP49 gene is a gene involved in DNA disorders as shown in Example 2, thereby strongly suggesting that TIP49 gene is a gene involved in DNA disorders not only in liver but also in any other tissues.

Though amino acid sequences somewhat differ among species, it has generally been considered that a homology of 30% to 40% and over exists among proteins having the same functions among different species. The existence of homology here is determined, as generally considered, by a calculating method which counts a homologous amino acid as positive; and when the length of amino acid chains differ from each other, the ratio of the part having a homology is indicated based on the length of the protein whose amino acid chain is shorter.

When TIP49 of a rat is compared with that of human, they differ from each other only in one amino acid on the amino acid level, thus TIP49 is a protein conserving the amino acid sequence very well between the species.

The functions of TIP49 in accordance with the present invention are characterized in that it forms a complex with TBP, has an ATPase activity, or has a DNA helicase activity. They can be confirmed, for example, by using the methods disclosed in Examples 7 to 9 in their respective cases.

This invention embraces proteins as variants of TIP49, insofar as they have the above-mentioned properties and comprises amino acid sequence modified by substitution, deletion or addition of one or plural (e.g., one to teens) amino acid(s) in the amino acid sequence set forth in SEQ ID NO.1 or NO.3 of the Sequence Listing.

A substitution, deletion, or addition of an amino acid can be effected, for example, by generating a mutation in a base sequence according to site-directed mutagenesis. Specifically, by using a synthetic oligonucleotide complementary to the DNA to be mutated, a part of the base sequence of DNA can be replaced with a desired sequence, or a desired sequence can be deleted or added. Further, when site-directed mutagenesis is repeated twice or thrice, a larger range of base sequence can be substituted, deleted, or added; or mutations can be generated at separated sites in a base sequence at the same time.

The substitution, deletion, or addition of the base sequence can also be effected by a method in which a gene is treated with a mutagen or a method in which a gene is selectively cleaved, a selected nucleotide is removed therefrom and/or added thereto, and then the gene is ligated.

The DNA or RNA coding TIP49 or TIP49 mutants of the present invention includes all the base sequences corresponding to any pattern of degeneracy.

With respect to RNA or the sense strand of DNA, there can be an antisense RNA or antisense DNA which consists of a sequence complementary to them respectively. In this specification, unless otherwise specifically mentioned, DNA or cDNA represents one which consists of double strands comprising the sense strand and the antisense strand; RNA represents one which consists of single strand; antisense DNA or antisense RNA represents one which consists of single strand.

The polynucleotides of this invention embrace DNA, RNA, and all of those to which one or more of nucleotides comprising a base, phosphoric acid, and sugar are bonded, including those which are not present in nature. The antisense polynucleotides can be similarly defined mutatis mutandis.

The antisense polynucleotides of this invention embrace all derivatives analogous to the polynucleotides in terms of their stereochemical structures or functions. For example, they may be polynucleotides 3'- or 5'-termini of which are bound to other substances, or may be those having undergone modifications such as substitution, deletion, or addition in at least any one of bases, sugars, and phosphoric acids of the polynucleotides, or those having bases, sugars or phosphoric acids not present in nature, or those having skeletons other than sugar-phosphoric acid skeletons.

The antisense polynucleotides or derivatives thereof may be ones that hybridize to any part of the coding region of a polynucleotide encoding TIP49 or TIP49 variants. It is preferable that they have base sequences complementary to a part of mRNA and can hybridize to the mRNA.

An antisense polynucleotide is believed to regulate expression of the protein by binding to DNA or mRNA that carries genetic information, and thereby influencing the normal flow of transmission of genetic information so as to inhibit biosynthesis of proteins at any of the following stages:
(1) transcription from the gene to pre-mRNA;
(2) processing from the pre-mRNA to mature mRNA;
(3) passing of the mature mRNA through nuclear membranes;
(4) translation of the mature mRNA to the protein.

For the ease of hybridization, it is considered to be advantageous that antisense polynucleotides or derivatives thereof are designed such that they have the base sequences complementary to the base sequence of a region which forms a stem loop of RNA. (Clinical Immunology, Vol. 25, p 1200-1206, 1993.)

It is also expected that polynucleotides having sequences complementary to sequences existing in the vicinity of the translation start codon or at a ribosomal binding site, a capping site or splicing site are generally provided with a great inhibitory effect on expression. (Cancer and Chemotherapy, Vol. 20, No. 13, p 1899-1907.)

Therefore it is expected that an antisense polynucleotide of the present invention or derivatives thereof, having a sequence complementary to sequence existing in the vicinity of the translation start codon of TIP gene or TIP coding mRNA, or at a ribosomal binding site, a capping site or splicing site, provides high inhibitory effect.

Derivatives that have enhanced activity in at least one of nuclease resistance, tissue selectivity, cell permeability, and binding strength are preferable among generally known derivatives. Especially preferable derivatives are polynucleotide derivatives which have phosphorothioate bonds as their skeleton structures. The antisense polynucleotides and derivatives thereof of this invention also embrace derivatives having these functions and structures.

For the method of producing the antisense polynucleotide or derivatives thereof of this invention, a common method can be used. For example, if the antisense polynucleotides of this invention are DNA or RNA of the natural type, they can be obtained either by synthesis using a chemical synthesizer or by the PCR method that employs the gene encoding TIP49 or a TIP49 mutant as a template. Also, among the derivatives, there are ones that can be synthesized using a chemical synthesizer (e.g., Type 394 available from Perkin Elmer Inc.), such as the methylphosphonate type and the phosphorothioate type. In this case, the synthesizing operations may be conducted according to the Manual attached to the chemical synthesizer and the synthesized products as obtained can be purified by one of HPLC methods using reverse phase chromatography or the like in order to produce the desired polynucleotides or derivatives thereof.

The whole length or a part of polynucleotides or antisense polynucleotides of this invention can be used as probes to detect TIP49 gene.

The number of kind of human protein is said to be 3 x 10⁹. Therefore, DNA of 16 bases which comes in 4¹⁶ types is considered to be able to recognize any human proteins individually. That is the length required of a probe is, theoretically, 16 bases. For practical purposes, a length longer than that is desirable. Practically, however, a length of 12 bases or more is often used.

Also, the region used as the probe may be a non-coding region or a coding region. The region whose GC content is 30 to 70% is preferable in view of the ease of hybridization because of its stereochemical structure.

The polynucleotides or antisense polynucleotides of this invention used for a probe can be detected, if they have been labeled. It is preferable to use, for instance, radioisotopes (RI), fluorescent materials (e.g.,FITC or rhodamine), enzymes, and avidin or biotin as labeling means. Especially, it is preferable to use RI.

Methods for detecting genes are, for instance, Northern blot hybridization, RT-PCR (Current Protocols in Molecular Biology (Greene Publishing Associates and Wiley-Juter Science), Chapter 15), and in situ hybridization (ibid. Chapter 14).

The optimum conditions for hybridization are different according to the length of the probe or a membrane used. That is, conditions for hybridization naturally have a certain range.

The Examples of the present invention disclose optimum conditions worked out in view of the nature of the membrane which were in the Examples and the length of the probe. If the membrane and the probe length differ, hybridization, needless to say, can be achieved under different hybridization conditions. For instance, hybridization may be effected in the absence of sodium pyrophosphate. A standard embodiment is as follows:
(1) Prehybridization conditions
   6 to 10 x SSC
   5 to 10 x Denhardt's solution
   Not more than 0.1% sodium pyrophosphate
   About 100 µg/ml thermally denatured DNA
   O.1 to 1% SDS
   Total liquid amount about 50ml
   Reaction temperature 35 to 37°C
   Reaction time 50 minutes to 70 minutes
(2) Hybridization conditions
   6 to 10 x SSC
   5 to 10 x Denhardt's solution
   Not more than 0.1% sodium pyrophosphate
   About 100 µg/ml thermally denatured DNA
   1 x 10⁶ to 2 x 10⁶ cpm/ml cDNA probe
   Reaction temperature 35 to 42°C
   Reaction time 12 to 20 hours

It has been well-known, when chemically synthesizing DNA or RNA, to perform a chemical modification such as methylating or biotinating a side chain or replacing O in a phosphate group with S. For example, when chemically synthesizing the DNA set forth in SEQ ID NO. 2 or 4 in the Sequence Listing, the above-mentioned chemical modification can be effected, so as to synthesize a DNA (derivative) different from the DNA listed in the Sequence Listing per se.

Therefore, the DNA and RNA of the present invention encompasses the above-mentioned chemically modified DNA, RNA, and antisense polynucleotides within their scopes. In the present invention, any chemically modified DNA or RNA can be used without any particular problem as long as it can exhibit a function of coding a protein or a function as a probe. Also, any chemically modified antisense polynucleotide can be used without any particular problem as long as it can exhibit a function of inhibiting the biosynthesis of a protein or a function as a probe. Here, labeling is included in the chemical modification.

With respect to the antigenicity of TIP49, this invention makes it clear that an antibody can be easily obtained by immunizing an animal except the animal from which said TIP49 origined, as illustrated in Example 5. Therefore, an antibody of this invention recognizing TIP49 embraces either of polyclonal antibody and antiserum which are obtained by immunizing an animal except the animal from which said TIP49 originated, and which can be confirmed to recognize TIP49 of this invention by Western blotting method ELISA method immunostaining method (e.g., measurement by FACS) and so on.

Also, as an immunogen it is often performed to use a part of protein or the part of protein to which any other carrier protein such as a bovine serum is attached. Said part of protein may be synthesized by a peptide synthesizer. Also, said part of protein preferably comprise 8 or more amino acids.

With respect to a substance whose antigencity was made public, it is well known that if a polyclonal antibody was obtained by sensitization, a monoclonal antibody is secreted by the hybridoma obtained from the lymphocytes of the sensitized animal (Chapter 6, Antibodies A Laboratory Manual, Cold Spring Harbor Laboratory Press,1988).Therefore, antibodies recognizing TIP49 of this invention embrace monoclonal antibodies in the scope.

In this invention, an antibody also embraces an active fragment thereof. An active fragment means a fragment of an antibody having activity of antigen-antibody reaction. Specifically named, there are active fragments, such as F(ab')₂, Fab', Fab, and Fv.

For example, F(ab')₂ results if the antibody of this invention is digested with pepsin, and Fab results if digested with papain. Fab' results if F(ab')₂ is reduced with reagent such as 2-mercaptoethanol and alkylated with monoiodoacetic acid. Fv is a mono active fragment that the variable region of heavy chain and the variable region of light chain are connected with a linker.

A chimeric antibody is obtained by conserving these active fragments and substituting the fragments of another animal for the fragments other than these active fragments.

Methods for detecting TIP49 embrace, for example, the use of an antibodies and the use of enzyme reaction.

Methods using antibodies are specifically (1) a method for detecting TIP49 by use of antibodies recognizing TIP49, and (2) a method for detecting TIP49 by use of antibodies recognizing TIP49 and labeled secondary antibodies to said antibodies. For instance, radioisotopes(RI), enzymes, avidin or biotin, and fluorescent materials(FITC or rhodamine) are available for labeling.

For a methods using enzyme reaction, for example, ELISA may be available.

### Examples

The present invention will further be explained with reference to the following examples, which would not restrict the present invention.

### (Example 1) Isolation of TIP49 Gene and Determination of Amino Acid Sequence of TIP49

### 1. Preparation of Rat Nuclear Extract

In conformity to the method described in "Bio-Manual Series 5, Transcription Factor studying method ," pp. 27-37 (Yodosha, 1993), 25 ml of rat liver cell nuclear extract was prepared.

Specifically, the following process was taken. Each operation was performed at 4°C.

The liver (20 g) was taken out from a rat, immersed in cool PBS, and then washed with PBS three times. Subsequently, tissues of the liver were cut into small pieces with scissors and transferred to a homogenizer. Thereafter, 60 ml of sucrose solution I (10 mM Hepes (pH 7.6), 15 mM KCl, 0.15 mM spermine, 0.5 mM spermidine, 1 mM EDTA, 2.2 M sucrose, 5% glycerin, 0.5 mM DTT, 0.5 mM PMSF, 14 µg/ml aprotinin) were added thereto and homogenized together. Into a centrifuge tube, 10 ml of sucrose solution II (10 mM Hepes (pH 7.6), 15 mM KCl, 0.15 mM spermine, 0.5 mM spermidine, 1 mM EDTA, 2 M sucrose, 10% glycerin, 0.5 mM DTT, 0.5 mM PMSF, 14 µg/ml aprotinin) were introduced. The homogenized sample was superposed thereon, and the resulting mixture was centrifuged at 24,000 rpm for 50 minutes. The sediment was suspended in 5 ml of nuclear solution (10 mM Hepes (pH 7.6), 100 mM KCl, 0.1 mM EDTA, 10% glycerin, 3 mM MgCl₂, 0.5 mM DTT, 0.1 mM PMSF, 14 µg/ml aprotinin), and the whole volume was measured. The absorbance of this liquid at 260 nm was measured, and the DNA concentration was adjusted to 0.5 to 1.0 mg/ml. KCl was added thereto such that the final concentration became 0.55 M. After being mixed slowly, the mixture was shaken for 15 minutes, so as to dissolve the nucleus. Subsequently, the mixture was centrifuged at 40,000 rpm for 60 minutes. The centrifuged supernatant was collected, and 0.3 g of ammonium sulfate was added thereto per 1 ml of the liquid, so as to effect ammonium sulfate precipitation. Then, the mixture was centrifuged at 35,000 rpm for 30 minutes. The supernatant was discarded, the sediment was dissolved in a small amount (0.2 to 0.5 ml) of dialysis liquid (25 mM Hepes (pH 7.6), 0.1 mM EDTA, 40 mM KCl, 10% glycerin, 1 mM DTT), and the solution thus obtained was further dialyzed with the above-mentioned dialysis liquid. After the completion of dialysis, centrifuge (at 12,000 rpm for 5 minutes) was effected again, the supernatant was collected, and its protein concentration was adjusted to 10 mg/ml by measuring the absorbance at 260 nm and 230 nm, so as to yield a nuclear extract. The nuclear extract thus obtained was conserved at -80°C until its use.

The protein was isolated from the rat liver cell nuclear extract on the basis of the method described in "Bio-Manual Series 5, Transcription Factor Studying Method," pp. 215-219 (Yodosha, 1993). In practice, the rat liver nuclear extract was used in place of the TFIID fraction.

### 2. Two-dimensional Electrophoresis

1) The above-mentioned rat liver nuclear extract was processed as being passed through a nickel column (namely, a prep column (manufactured by Bio-Rad Laboratories) which contained 1 ml of Ni-NTA agarose (manufactured by Quiagen Ltd.)), 800 µl of the extract thus processed and 240 µl of HXm TBP-C (C-terminal protein of mouse TBP having a tag which consisted of 6 histidine residues linked to each other (see Nucleic Acids Res. 22, 1179-1185 (1994)) were reacted at 4°C for 12 hours. Thereafter, 80 µl of 50%-slurry nickel NTAP agarose (manufactured by Quiagen Ltd.) was added to the reaction liquid, in which the reaction was allowed to proceed at 4°C for an hour.
2) The reactant (gel) generated above was collected, washed with NP 0.1 (20)(25 mM HEPES/KOH (pH 7.9), 10% glycerol, 0.1 M KCl, 0.5 M NaCl, 0.1% NP-40, 20 mM imidazolehydrochloric acid (pH 7.9), 1 mM PMSF, 0.5 mM 2ME) buffer, and then was eluted with 100 µl of 8 M urea solution (8 M urea, 0.1 M NaH₂PO₄, 0.01 M Tris) for 20 minutes. This process was repeated four times.
3) The eluate, with 1/4 volume of TCA solution (100% (w/v) TCA, 4 mg/ml deoxycholic acid) added thereto, was left for 30 minutes at 4°C and then was centrifuged at 15000 rpm for 30 minutes.
4) After the centrifuge, the supernatant was removed, and the sediment was washed three times with 1 ml of 100% acetone.
5) After acetone was removed as being aspirated with a vacuum pump, the sediment was suspended with 5 µl of 8 M urea solution and 1 µl of 20 sample solution (1.7% SDS, 3.3% Triton X-100, 2.4 M 2-mercaptoethanol, 1/3 volume Bio-Lyte 3/10 (manufactured by BIO-RAD)), and the suspension thus obtained was mounted as a sample on a tube gel for isoelectric focusing.
6) On the sample, 5 µl of sample protecting solution (2% Triton X-100, 1/20 volume Bio-Lyte 3/10) were superposed. While using 0.1 N NaOH solution and 0.06% H₃PO₄ solution as the upper and lower electrophoresis solutions, respectively, electrophoresis was effected for 10 minutes at 400 V and for 45 minutes at 800 V.
7) After the electrophoresis, the gel was taken out, immersed in SDS reducing solution (125 mM Tris/HCl (pH 6.8), 10% glycerol, 2% (w/v) SDS, 0.72 M 2-mercaptoethanol, 0.00125% (w/v) Bromophenol Blue) for 10 minutes, and then was subjected to electrophoresis at 35 mA for 3 hours on a slab gel for SDS-PAGE.
8) After the SDS-PAGE, the transfer of the protein from the gel to a membrane (manufactured by Millipore) was performed by a conventional method using an electroblotting apparatus (manufactured by Nihon Eido). Thereafter, the protein was stained with Coomassie Brilliant Blue.

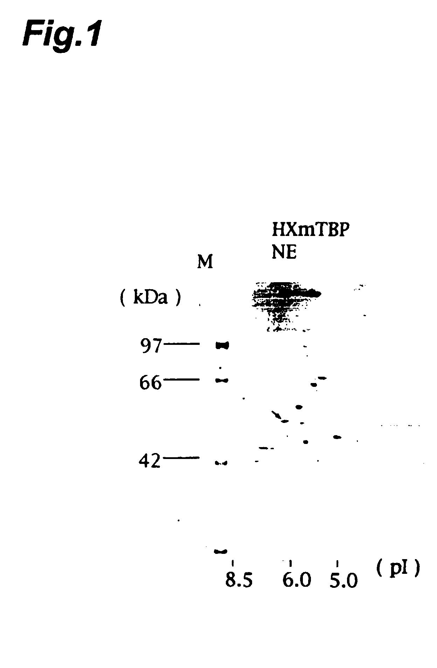

Fig. 1 shows the results of the two-dimensional electrophoresis.

### 3. Determination of Partial Amino Acid Sequence in Rat Nuclear Extract

The part of each dot transcribed to the membrane was cut with a cutter knife, the part thus cut was digested with lysyl peptidase, and then fractionated into individual peptide fragments by high-performance liquid chromatography, one of which was subsequently analyzed with a protein sequencer (manufactured by Beckman). From the dots indicated by arrows in Fig. 1, four peptide fragments made of unknown amino acid sequences (the following sequences (1) to (4)) were obtained.
(1) MKIEEVKSTTK
(2) QAASGLVGQENA
(3) EVYEGEVTEL
(4) ILADQQD

### 4. Synthesis of DNA Probe Corresponding to Protein in Rat Liver Cell Nuclear Extract

1) Using a DNA synthesizer (manufactured by ABI), a DNA probe whose sequence represented by the following sequence (5) or (6) and a DNA probe whose sequence represented by the following sequence (7) were synthesized according to the above-mentioned amino acid sequences (4).
(5) GCIGATCAIC AIGATCGITA CATG
(6) GCICATCAIC AIGATAGRTA CATG
(7) GAIGTITATG AIGGIGAIGT

Here, A, T, G, C, I, and R represent bases which are adenine, thymine, guanine, cytosine, inosine, and adenine or guanine, respectively.
2) The DNA probes represented by the above-mentioned sequences (5) to (7) were labeled with Takara MEGA LABEL Kit (trademark; manufactured by Takara Shuzo Co., Ltd.) in conformity to its instruction manual.
3) Then, a DNA reaction liquid having the following composition was prepared, incubated at 37°C for 30 minutes, and then heat-treated at 70°C for 10 minutes, so as to deactivate enzymes.

| | |
|---|---|
| DNA probe (2 pmol/µl) | 3 µl |
| 10x phosphorylation buffer | 3 µl |
| [γ-³²P]ATP (370 MBq/ml) (manufactured by Amersham) | 5 µl |
| T4 polynucleotide kinase | 1 µl |
| | total 10 µl |

4) Sephadex G-25 (trademark; manufactured by Pharmacia AB) equilibrated with T50E (50 mM Tris-Cl pH 8.0, 1 mM EDTA) was charged into a 1.5-ml poly-prep column (manufactured by Bio-Rad Laboratories, Inc.) such as to yield a bed volume of 1 ml, and the DNA reaction liquid heat-treated in 3) was mounted on the column.
5) Thereafter, 200 µl of T50E was caused to flow through the column four times, and the fraction eluted in the second 200 µl was used as a DNA probe fraction in the subsequent operations.

### 5. Acquisition of Gene from Rat Liver cDNA Library Using DNA Probe Corresponding to Protein in Rat Liver Cell Nuclear Extract

1) A Wistar-line rat liver cDNA library was prepared according to a conventional method by using Timesaver cDNA Synthesis Kit (trademark; manufactured by Pharmacia AB). By using this cDNA library, plaques on the order of 10⁶ were prepared on a NZY agar culture, This cDNA library was fixed onto a nitrocellulose membrane (manufactured by Millipore Corp.) according to the method described in "Molecular Cloning Second Edition" (Cold Spring Harbor Laboratory Press, 1989), Chapter 9. Then, this membrane was washed with 2 x SSC at 60°C.
2) Of the labeled DNA probe fraction obtained by 4., 150 µl were hybridized with the cDNA probe fixed on the membrane under the following conditions.
   (1) Prehybridization
      6 x SSC
      5 x Denhaldt's
      0.05% sodium pyrophosphate
      100 µl/ml denatured herring sperm DNA
      0.5% SDS
      total liquid amount: 50 ml
      reaction temperature: 37°C
      reaction time: 1 hr
   (2) Hybridization
      6 x SSC
      1 x Denhaldt's
      0.05% sodium pyrophosphate
      100 µl/ml denatured herring sperm DNA
      1 x 10⁶ cpm/ml cDNA probe
      total liquid amount: 50 ml
      reaction temperature: 42°C
      reaction time: 18 hr
3) The nitrocellulose membrane on which the hybridization was finished was washed under each of the following conditions.
   (1)
      6 x SSC, 0.1% SDS: 500 ml
      temperature: 40°C
      time: 20 min
   (2)
      3 x SSC, 0.1% SDS: 500 ml
      time: 42°C
      time: 20 min
4) The washed nitrocellulose membrane was exposed to KODAK XAR5 film at -80°C for one night, so as to yield an autoradiograph.
5) From the autoradiograph thus obtained, positions of positive plaques were determined, and their corresponding plaques on the agar were collected into an SM solution (0.1 M NaCl, 0.16 M MgSO₄, 50 mM Tris-HCl (pH 7.5), 0.01% gelatin).
6) The collected plaques were caused to form further plaques on an NZY agar culture again by a conventional method, and fixed onto a nitrocellulose membrane.
7) The steps of 2) to 6) were repeated three times. As a result, the positive plaques became a single plaque. This plaque was collected, and then suspended in 100 µl of SM solution, so as to stabilize phages. The gene isolated from this plaque was named TIP49 gene.

### 6. Large-scale preparation of TIP49 Gene

1) 50 µl of phages suspended in the SM solution and 20 µl of Y1090r-Escherichia coli were mixed together, and the mixture was left for 15 minutes at 37°C.
2) Thereafter, the solution mixed in 1) was transferred to 10 ml of NZY culture containing 100 µg/ml of ampicillin and cultured for 6 hours at 37°C.
3) After the culture was centrifuged at 8000 rpm for 5 minutes, the supernatant was collected.
4) Into the supernatant, 1 ml of 5 M NaCl and 1.1 g of polyethylene glycol 6000 were added and dissolved.
5) The resulting solution was left for an hour on ice, and then was centrifuged at 10000 rpm for 20 minutes at 4°C.
6) The sediment was collected and then suspended in 700 µl of SM solution.
7) The resulting suspension, with 500 µl of chloroform added thereto, was stirred, so as to dissolve the remaining Escherichia coli.
8) The solution thus obtained was centrifuged at 5000 rpm for 10 minutes, and the aqueous layer was collected.
9) To the collected aqueous layer, 1 µl each of 1 mg/ml RNase A and 5 mg/ml of DNase I (both manufactured by Sigma) was added. After the mixture was left for an hour at 37°C, 600 µl of 20% polyethylene glycol 6000 (0.8 M NaCl) was added thereto, and the resulting product was left on ice for 30 minutes.
10) After this product was centrifuged at 15000 rpm for 20 minutes at 4°C, the sediment was collected.
11) To this sediment, 500 µl of SM solution, 50 µl of 5 M NaCl, and 50 µl of 0.5 M EDTA were added. Thereafter, with 400 µl of phenol added thereto, the mixture was stirred, so as to dissolve phages and liberate cDNA.
12) After the solution was centrifuged at 15000 rpm for 5 minutes at room temperature, the aqueous layer was collected. The aqueous layer thus collected , with 1 ml of ethanol added thereto, was centrifuged for 20 minutes at 15000 rpm, and then the liquid layer was discarded.
13) The sediment was washed with 1 ml of 70% ethanol, and dissolved in 100 µl of TE solution (Tris-Cl pH 8.0 10 mM, 1 mM EDTA), so as to yield a DNA solution.

### 7. Insertion of TIP49 Gene into Vector

1) The following system for cutting DNA was made, and DNA was cut with restriction enzyme EcoRI (manufactured by Takara Shuzo Co., Ltd.).

| | |
|---|---|
| DNA solution (prepared in 6.) | 20 µl |
| EcoRI (manufactured by Takara Co., Ltd.) | 2 µl |
| RNase A (manufactured by Nippon Gene Co., Ltd.) | 1 µl |
| 10 x H buffer (manufactured by Takara Shuzo Co., Ltd.) | 10 µl |
| sterilized water | 67 µl |
| | total 100 µl |
| reaction temperature: 37°C reaction time: 4 hr | |

2) Thereafter, electrophoresis with 0.7% NuSieve GTG agarose (trademark; manufactured by Takara Shuzo Co., Ltd.) was effected, DNA near 1.6 kbp was cut out, and this DNA was collected by using GENE CLEAN II (trademark; manufactured by Funakoshi Co, Ltd.) in conformity to the instruction manual thereof.
3) After pBluescript II (manufactured by Stratagene) for integrating DNA was cut with EcoRI, dephosphorylation was effected.
(1) The cutting with EcoRI was effected in the following system.

| | |
|---|---|
| pBluescript II (1 µg/µl) | 3 µl |
| 10 x H buffer | 2 µl |
| EcoRI | 2 µl |
| sterilized water | 13 µl |
| | total 20 µl |
| reaction temperature: 37°C reaction time: one night | |

(2) Subsequently, with 2 µl of 1 M Tris pH 8.0 and then 1 µl of Bacterial Alkaline Phosphatase (manufactured by Takara Shuzo Co., Ltd.) added thereto, the reactant was left for an hour at 65°C, so as to be dephosphorylated.
(3) Thereafter, phenol/CHCl₃ extraction was effected twice according to a conventional method, so as to deactivate enzymes. Then, the reactant was purified with ethanol precipitation, and the purified product was dissolved into TE solution at 100 µg/µl.
(4) The DNA obtained in 2) and the pBluescript II obtained in (3) were reacted in the following system, so as to insert the DNA into a vector.

| | |
|---|---|
| DNA (1.6 kbp; prepared in 2)) | 5 µl |
| pBluescript II NotI-cut product (prepared in (3)) | 1 µl |
| 10x ligation buffer (manufactured by Nippon Gene Co., | 2 µl |
| T4 ligase (manufactured by Nippon Gene Co., Ltd.) | 1 µl |
| sterilized water | 11 µl |
| | total 20 µl |
| reaction temperature: 16°C reaction 2 hr | |

### 8. Introduction of TIP49 Gene into Escherichia Coli

According to a conventional method, the whole reaction liquid was mixed with E. coli JM109 competent cell (manufactured by Takara Shuzo Co., Ltd.). The resulting mixture was reacted for 30 minutes on ice, for 45 seconds at 42°C, and then for 3 minutes on ice. Subsequently, with 900 µl of SOC culture added thereto, the mixture was left for an hour at 37°C, so as to introduce the vector into E. coli JM109. Thereafter, E. coli JM109 was collected.

The recombinant Escherichia coli into which TIP49 gene had been introduced was deposited in National Institute of Bioscience and Human-Technology, Agency of Industrial Science and Technology, Ministry of International Trade and Industry (1-3, Higashi 1-chome, Tsukuba-shi, Ibaragi-ken, postal code 305-8566, Japan) on June 23, 1997 (accession number: FERM P-16282), and then transferred to international deposit on May 25, 1998 (accession number: FERM BP-6377).

### 9. Determination of Base Sequence of TIP49 Gene

1) E. coli JM109 collected in 8. was sprinkled on an LB agar culture (containing 100 µg/ml ampicillin, 0.1 mM IPTG, and 0.004% X-gal) and was cultured for 16 hours at 37°C.
2) Of the colonies thus formed, white ones were planted on a 2 ml LB culture (100 µg/ml ampicillin) and then were cultured for 16 hours at 37°C.
3) Subsequently, the culture was centrifuged at 12000 rpm for one minute, so as to be condensed. And a plasmid DNA solution was collected according to Magic Miniprep (trademark; manufactured by Promega Corp.).
4) By using Dye Terminator FS Sequence Kit (manufactured by Perkin-Elmer, Inc.) and autosequencer ABI3739, the DNA thus collected was sequenced, whereby the whole base sequence was determined. The results are shown in SEQ ID NO. 2 in the Sequence Listing.

### 10. Determination of Amino Acid Sequence of TIP49

From the base sequence determined in 9., the amino acid sequence of TIP49 was determined. The results are shown in SEQ ID NO. 1 in the Sequence Listing.

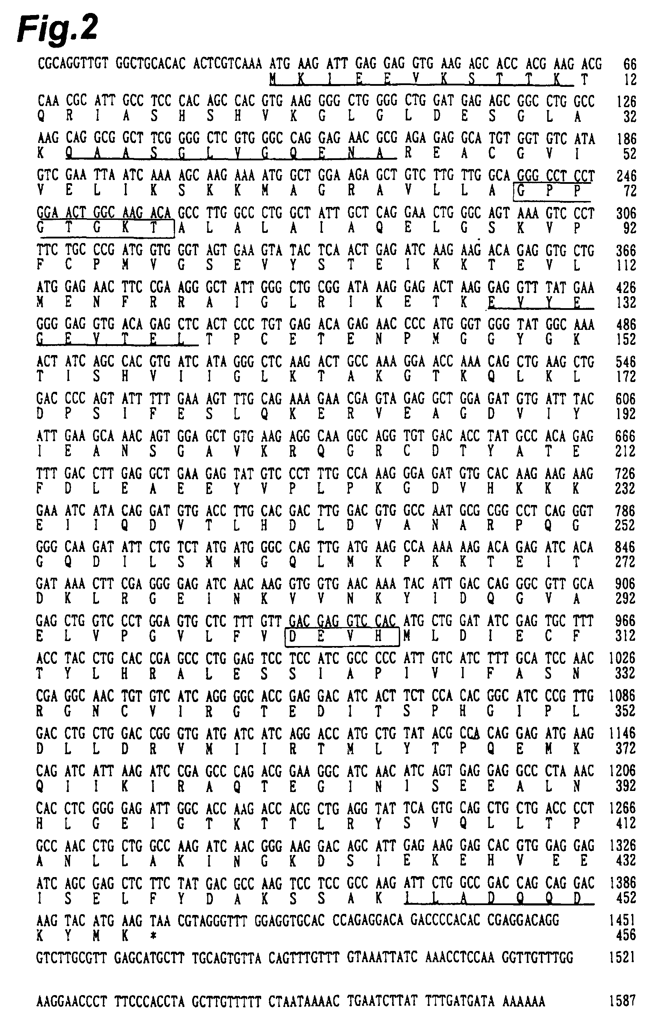

Also, Fig. 2 shows the amino acid sequence of TIP49 protein and the base sequence of TIP49 gene. In Fig. 2, the underlined sites refer to those of sequences (1) to (4) peptide-sequenced in 3.

On the other hand, the framed sites refer to those of sequences characteristic to ATPase and DNA helicase.

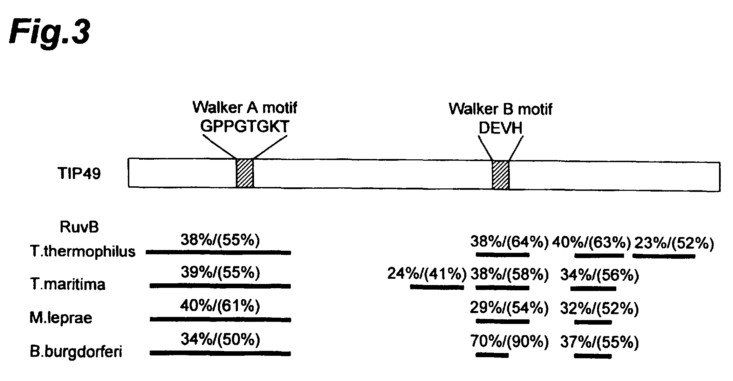

It was further found that TIP49 protein had a high degree of homology to RuvB protein expressed in procaryote. RuvB protein is a protein which recombines and repairs DNA. Fig. 3 shows the homology between TIP49 protein and RuvB protein as well as the homology between TIP 49 gene and RuvB gene. The values in parentheses in the figure refer to homology of the proteins, whereas the values without parentheses refer to homology of the genes. Conventionally, there have hardly been examples in which proteins originated from animal cells have a high degree of homology to proteins of procaryote.

Also, as for the sites bound to the probes (5) to (7) used in 4., it is presumed that (5) and (6) are sequence sites of the complementary strand of No. 1372 to No. 1395 in the base sequence, whereas (7) is a sequence site of the complementary strand of No. 415 to No. 434.

### (Example 2) Confirmation of Expression of TIP49 in Rat Liver Cancer Carcinogenesis State

### 1. Preparation of Various Kinds of Rat Livers

1) Ten 5-week-old Wistar-line rats were purchased; DEN (diethyl nitrosoamine), which was a DNA-disordering chemical, was intraperitoneally administered to seven of the rats at a concentration of 0.2 mg/g rat; these rats were killed one by one at 12, 24, and 48 hours thereafter; and their livers were collected and conserved in liquid nitrogen.
2) Two weeks later, continuous oral administration (once/day) of feed containing 0.02% of AAF (aminoacetylfluorene) to the remaining DEN-administered rats was started. The three rats with no DEN administration were subjected to a regenerating liver operation, and their livers were collected 12, 24, and 48 hours thereafter and conserved in liquid nitrogen.

### 2. Preparation of RNA

RNA of each rat liver was prepared according to the guanidine thiocyanate method described in "Molecular Cloning Second Edition" (Cold Spring Harbor Laboratory Press, 1989), pp. 7.3-7.84.

In practice, 1 g of each rat liver was pulverized in liquid nitrogen, added to 50 ml of 5.5 M guanidine thiocyanate solution, and homogenized therewith. Thereafter, ultracentrifugation was effected in conformity to the protocol, and further purification was effected, so as to yield an RNA specimen.

The RNA thus obtained specimen was purified into polyA RNA by using oligotex-dT3 super (trademark; manufactured by Takara Shuzo Co., Ltd.) according to its product manual.

### 3. Adjustment of Probe DNA

By using TIP49 gene, a probe for hybridization was made. In its making method, TIP49 gene mounted on pBluescript II was subjected to restriction enzyme processing so as to cut out the TIP49 gene portion. After the aimed gene was isolated by agarose gel electrophoresis, GENE CLEAN II (trademark; manufactured by Funakoshi Co., Ltd.) was used for purifying TIP49 gene. With 100 ng of TIP49 gene thus purified, a DNA probe was made by using Random Primed DNA Labeling Kit (manufactured by Boehringer-Mannheim GmbH) according to its product instruction manual with α-P³²dCTP (manufactured by Amersham International plc) and used for the next Northern blot hybridization.

### 4. Northern Blot Hybridization

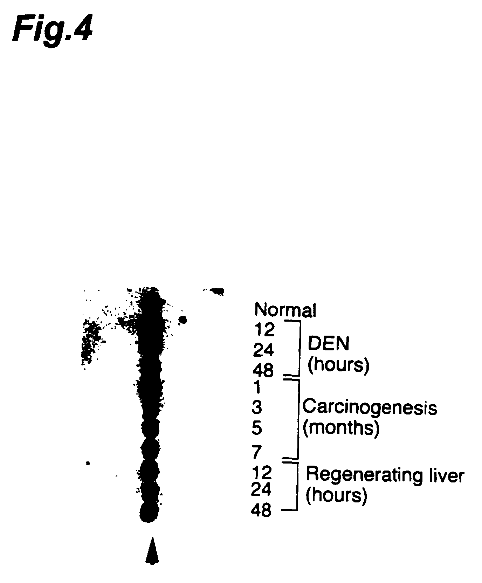

1) According to the Northern blot method (formaldehyde method) described in "Molecular Cloning Second Edition" (Cold Spring Harbor Laboratory Press, 1989), pp. 7.3-7.84, Northern blot hybridization was effected. However, the DNA probe made in 3 above was used.
   As for the RNA at the time of sample preparation, 300 ng of polyA RNA was used.
   The exposure time for autoradiography was set to 48 hours.
2) The TIP49 gene expression pattern shown in Fig. 4 was obtained by the operations of 1. to 4. 1).
   From Fig. 4, it was found that only the DEN-administered rat livers yielded dots denser than those of normal rat livers (i.e., increased the amount of mRNA expression). Since DEN was a substance inducing DNA disorder, it was found that TIP49 gene was a gene which increased at the time of DNA disorder. Therefore, it was assumed that there is a possibility of DNA disorder being detected by use of TIP49 gene.

### (Example 3) Confirmation of Intertissue Distribution of TIP49 Gene

For confirming the intertissue distribution, Rat MTN Blot (trademark; manufactured by Clonetech Laboratories, Inc.) was used. This product was a membrane, blotting the polyA RNA of each rat tissue, commercially sold for effecting the above-mentioned Northern blot hybridization. With this membrane, Northern blot hybridization was effected in a manner similar to Example 2. The exposure time for autoradiography was set to 96 hours.

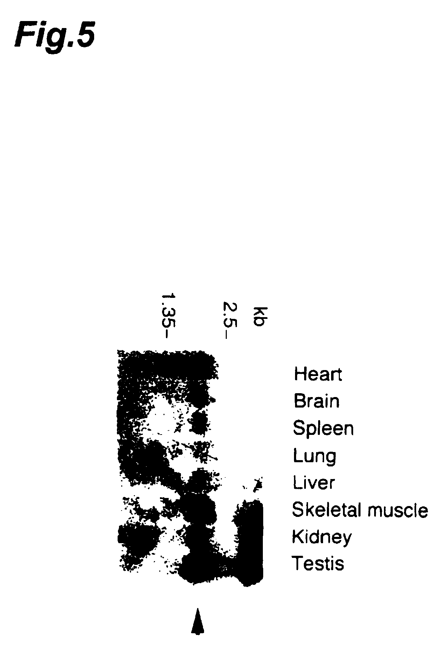

The results are shown in Fig. 5. As can be seen from these results, TIP49 gene was found to be a universal gene expressed in all the tissues while being strongly expressed at testis, skeletal muscle, heart, and kidney.

### (Example 4) Large-scale preparation of TIP49

### 1. Large-scale preparation of TIP49 Gene

### 1) Preparation of Recombinant Vector

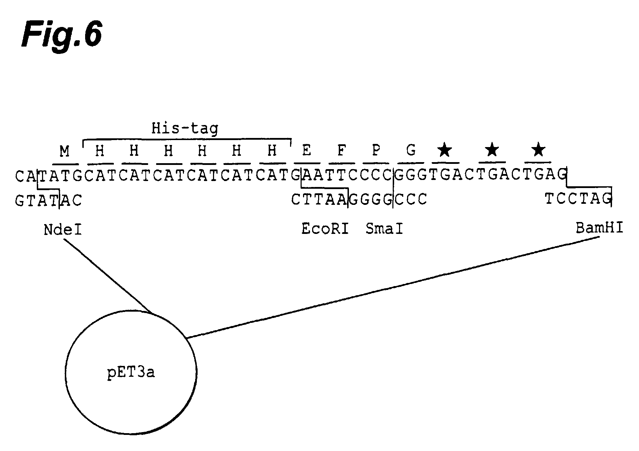

TIP49 gene was integrated into pET3a-His vector (manufactured by Invitrogen Corp.) shown in Fig. 6 in which a histidine tag had been introduced. The details thereof will be described in the following.
(1) First, the pBluescript II vector inserted with the TIP49 gene prepared in 7. of Example 1 was subjected to PCR method (described in "Current protocols in molecular biology" (Greene Publishing Associates and Wiley-Interscience, 1987), chapter 15), whereby the TIP49 gene part was amplified with the primers having the base sequences of the following sequences (8) and (9):
   sequence (8) CGAATTCATG AAGATTGAGG AGGTGAAGAG CACC
   sequence (9) CATACCCACC CATGGGGTTC TCTGTCTCAC

   The seven bases from the 5'-terminal of the sequence (8) constitute an EcoRI site.
(2) The amplified gene was cut with NcoI and EcoRI.
(3) Separately, the part existing on the C-terminal side of TIP49 gene on pBluescript II prepared in 7. of Example 1 was cut with EcoRI, so as to cut out a gene.
(4) The gene cut in (2) and the gene cut in (3) were ligated by using Ligation Pack (manufactured by Nippon Gene Co., Ltd.) according to its instruction manual, so as to make TIP49 gene.
(5) The TIP49 gene prepared in (4) was integrated into the EcoRI site of the pET3a-His vector into which the histidine tag had been introduced (Fig. 6).

### 2) Large-scale preparation of TIP49 Gene

Plasmid integrated with TIP49 gene was prepared in large-scale by the method described in "Molecular Cloning Second Edition" (Cold Spring Harbor Laboratory Press, 1989), chapter 1. The plasmid was introduced into JM109 competent cell (manufactured by Takara Shuzo Co., Ltd.) according to its instruction manual, and cultured for one night in an 800 ml LB culture containing ampicillin. Then, the plasmid was recovered from the collected JM109 according to the alkali method (described in "Molecular Cloning Second Edition" (Cold Spring Harbor Laboratory Press, 1989), 1.33-1.43). Here, the ultracentrifugation described in the above-mentioned publication was used three times.

### 2. Expression of TIP49

(1) The plasmid prepared in large-scale was introduced into Escherichia coli BL21 (DE3) pLysS.
(2) This Escherichia coli was cultured in an LB culture medium containing 100 µg/ml of ampicillin and, at the point in time when turbidity became 0.5 at a wavelength of 600 nm with a spectrophotometer (manufactured by Beckman), IPTG was added thereto such as to become 0.5 mM, and the expression of TIP49 was induced. As a control, the case without IPTC addition was also effected.

### 3. Purification of TIP49

(1) Two hours later, Escherichia coli was collected, suspended in Lysis Buffer, and then sonicated at 4°C. Thereafter, by using Beckman Optima XL-80, it was centrifuged with 50.2 Ti rotor at 1800 rpm for 15 minutes at 4°C.
(2) Thereafter, the supernatant was taken, and 1 M imidazole (pH 7.5) was added thereto until the final concentration became 10 mM. Then, it was purified with Ni-NTA agarose column (manufactured by Quiagen) according to its instruction manual under a non-denaturing condition.
   Separately, TIP49 was purified with liquid chromatography [base buffer (20 mM Tris-HCl (pH 7.9), 10% glycerol, 5 mM 2-ME, 0.5 mM PMSF), eluate (KCl with a gradient of 0.04 to 0.4 M)] using ion exchange chromatography MonoQ (trademark; manufactured by PharMingen) in conformance with the product manual.
(3) The purified specimen was subjected to 10% SDS-PAGE electrophoresis and then was stained with Coomassie Brilliant Blue.

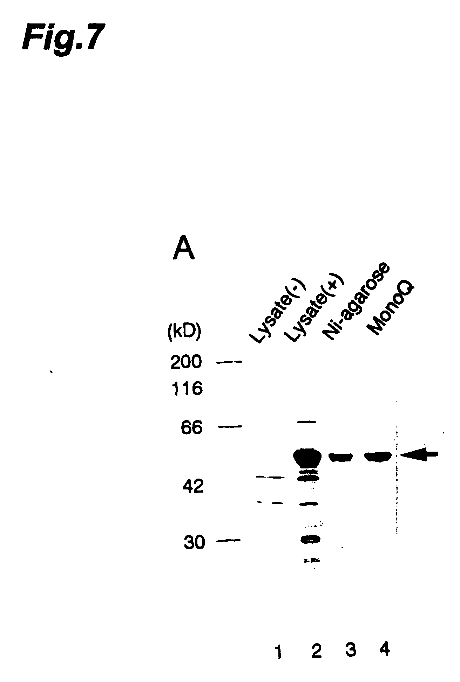

Fig. 7 shows the results of purification with Ni-NTA agarose column. In Fig. 7, lysate(-) refers to the electrophoresis of cell lysate without IPTC addition, whereas lysate(+) refers to the electrophoresis of cell lysate with IPTC addition. A band of TIP49 was confirmed at a position of 49 kD in each of Ni-NTA agarose column and MonoQ.

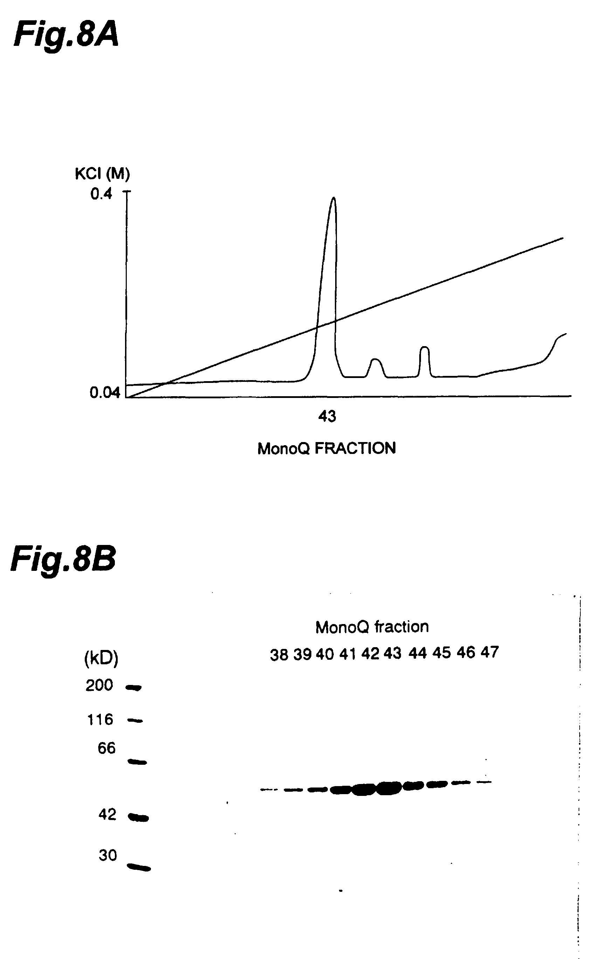

Also, Figs. 8A and 8B show the column pattern of MonoQ and results of electrophoresis (Coomasie staining) of purified protein. Fig. 8A shows the elution pattern, whereas Fig. 8B shows the results of electrophoresis of purified protein. It was found that TIP49 was eluted at a KCl concentration of about 0.2 M (fraction No. 43).

### (Example 5) Making of Anti-TIP49 Antibody

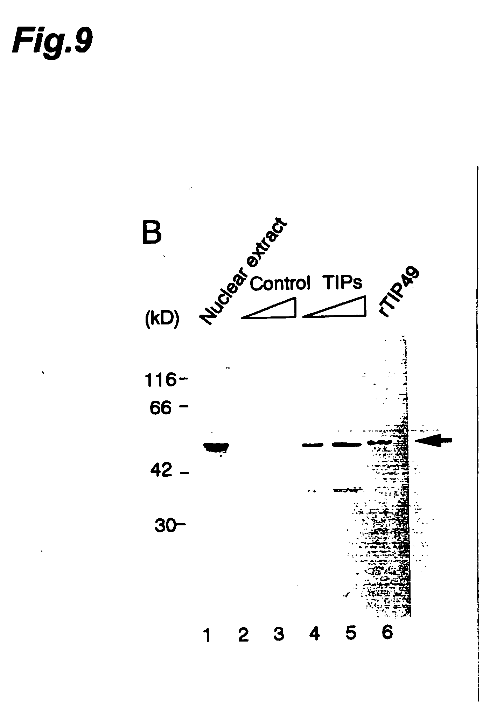

(1) For making the antibody, according to the method described in "Antibodies A Laboratory Manual" (Cold Sprong Harbor Laboratory, 1988), chapter 5, a rabbit was immunized with 1 mg of the purified TIP49 obtained in Example 4, and antiserum was collected.
(2) For each of a rat liver cell nuclear extract, TIP49 purified from the nuclear extract, and the recombinant TIP49 purified with the Ni-NTA agarose column in Example 4, analysis was effected according to Western blot method using the anti-TIP49 antibody. Fig. 9 shows the results. In Fig. 9, "Nuclear extract" refers to the lane of nuclear extract, whereas "TIPs" refers to TIP49 purified from the nuclear extract. "Control" is a band of beads of Ni-NTA agarose column after TIP49 was eluted. A band of TIP49 was confirmed at a position of 49 kD. "rTIP49" is the band of recombinant TIP49, whose molecular weight has increased by the amount of histidine tag. As a result, an antigen-antibody reaction was confirmed, and it was also confirmed that the TIP49 purified from the nuclear extract and the biotechnologically expressed TIP49 were the same.

### (Example 6) Confirmation of TIP49 Forming Complex with TBP

TBP with an HA tag was expressed within an animal cell, and a test for confirming that TIP49 formed a complex with TBP was effected.

### 1. Making of Nuclear Extract

1) HA-TBP gene was introduced into mouse cell strain FM3A (The Institute of Physical and Chemical Research Gene Bank RCB0086) with retrovirus vector, so as to make recombinant FM3A cells.
2) The recombinant FM3A cells were prepared such as to become 7 x 10⁵/ml in 1 l of a culture in which FCS had been added to an ES culture (manufactured by Nissui Co., Ltd.) to yield a concentration of 10%.
3) According to the method described in "Bio-Manual Series 5, Transcription factor studying method," pp. 27-37 (Yodosha, 1993), 4.4 ml (3.5 mg/ml) of nuclear extract were prepared from the cultured FM3A. Specifically, after 1 liter of FM3A culture liquid was centrifuged and the cells were collected therefrom, the preparation was performed in conformance with the condition described in "Bio-Manual Series 5, Transcription Factor Studying Method," pp. 27-37 ( Yodosha, 1993).

### 2. Confirmation of TIP49-TBP Complex Formation

1) Anti-HA antibody (manufactured by BAbCo) was bound to protein A Sepharose beads (manufactured by Pharmacia AB), so as to make anti-HA antibody/beads.
2) The nuclear extract made in 1. and anti-HA antibody/beads were mixed together, and immunoprecipitation was effected according to the method described in "Antibodies A Laboratory Manual," chapter 11.
3) The beads precipitated in 2) were eluted with an IP buffer (containing 1 mg/ml of HA peptide).
4) For the FM3A nuclear extract (N.E.), the supernatant (IP sup.) obtained by centrifuging the beads immunoprecipitated in 2), and the eluate (Elute) produced in 3), by using anti-TBP antibody, anti-HA antibody, and anti-TIP49 antibody, respectively, analysis by Western blot method in conformance with the method described in "Current protocols in molecular biology" (Greene Publishing Associates and Wiley-Interscience, 1987), chapter 1 was effected.

Specifically, the analysis by Western blot method was effected by the following Western blotting. Namely, the gel which had been subjected to SDS-PAGE and thereafter immersed in a transfer buffer (125 mM Tris-base, 960 mM glycine, 20% methanol) and a PVDF membrane (manufactured by Millipore Corp.; Immobilon) which has been immersed in methanol and thereafter immersed in the transfer buffer were held between two sheets of 3mm filter paper (manufactured by Whatman, Inc.) which had been immersed in the transfer buffer, and transfer of protein to a membrane was effected for 60 minutes at 170 mA by using a flat type transfer apparatus (manufactured by Nihon Eido Co., Ltd.). The membrane to which protein was thus transfered was blocked with 3% skim milk (manufactured by Snow Brand Milk Products Co., Ltd.)/TBST (20 mM Tris-Cl pH 7.5, 150 mM NaCl, 0.05% Tween 20). Anti-TBP antibody, anti-HA antibody, and anti-TIP49 antibody were used as the primary antibody, whereas alkaline-phosphatase-labeled anti-rabbit IgG antibody (manufactured by Promega Corp.) was used as the secondary antibody. For the coloring reaction, ProtoBlot Western Blot AP System (manufactured by Promega Corp.) was used.

Each antibody was reacted with the secondary antibody labeled with alkaline phosphatase, and alkaline phosphatase was reacted with a substrate (NBT, BCID (both manufactured by Promega Corp.)), whereby each band was colored on the gel.

The anti-TBP antibody was produced as immunizing a rabbit according to the method described in "Antibodies A Laboratory Manual," chapter 5.

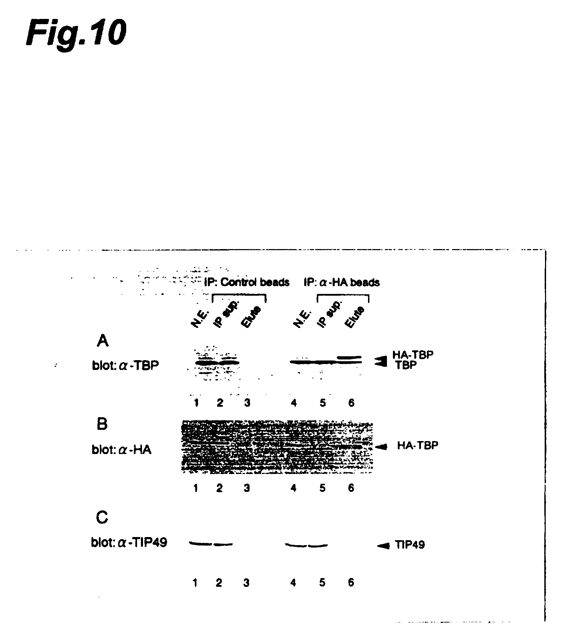

Fig. 10 shows the results. In Fig. 10, "N.E." (in lanes 1 and 4) represents the lane of FM3A nuclear extract, "IP sup." (in lanes 2 and 5) represents the supernatant at the time of immunoprecipitation, and "Elute" (in lanes 3 and 6) represents the eluate of immune precipitate. The upper stage (a), middle stage (b), and lower stage (c) in Fig. 10 represent the results obtained when the anti-TBP antibody was used as the primary antibody, when the anti-HA antibody was used as the primary antibody, and when the anti-TIP49 antibody was used as the primary antibody, respectively. In the rightmost lane (lane 6) in Fig. 10, lanes of TBP and TIP49 were seen, whereby it could be confirmed that TIP49 and TBP formed a complex and immunoprecipitated with the anti-HA antibody/beads.

### (Example 7) Confirmation of ATPase Activity of TIP49 #1

1) The eluate of each of fractions No. 38 to No. 47 purified with MonoQ and ATP (γ-³²P ATP) in which phosphorus (P) in γ site had been labeled as ³²P were reacted for 60 minutes at 37°C in a reaction liquid having the following composition:
   20 mM Tris/HCl
   70 mM KCl
   2.5 mM MgCl₂
   1.5 mM dithiothreitol (DTT)
   0.1 mM ATP
   γ-³²P ATP 0.125 µl
   SP (sterilized distilled water) to yield a total volume of 20 µl

   Separately, M13 ssDNA was added so as to effect the above-mentioned reaction.
2) To the reaction liquid, 100 µl of 0.1 N HCl, 100 µl of 2 mg/ml BSA, and 250 µl of 10% (W/V) active carbon were added. The resulting mixture was stirred and then allowed to stand for 15 minutes on ice.
3) Thereafter, the mixture was centrifuged at 4°C for 15 minutes at 15000 rpm, and 200 µl of the resulting supernatant were dispensed into a new tube.
4) This supernatant was centrifuged at 4°C for 5 minutes at 15000 rpm, and 100 µl of the resulting supernatant were dispensed into a new PCR tube (manufactured by Perkin-Elmer, Inc.).
5) The concentration of radiation in this supernatant was measured with a liquid scintillation counter (manufactured by Beckman, Inc.) according to its product manual.

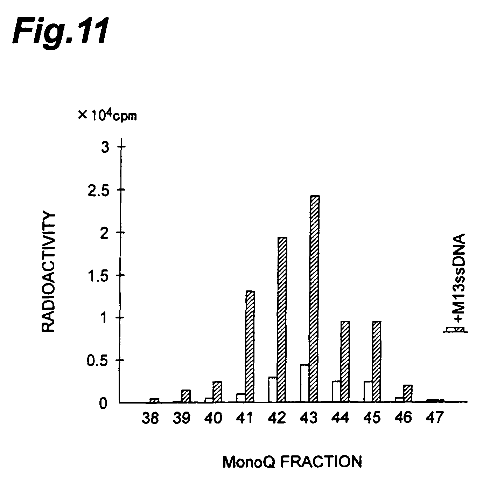

The results are shown in Fig. 11. It can be seen that ³²P in γ site of ATP was cut and liberated by TIP49, so that the radiation thereof was measured. In Fig. 11, whitened bars refer to the case without the M13 ssDNA addition, from which it has been found that the liberated ³²P depends on the amount of TIP49 in the reaction liquid. Also, it has been found that the ATPase activity of TIP49 is enhanced by the addition of M13 ssDNA.

### (Example 8) Confirmation of ATPase Activity of TIP49 #2

The TIP49 (fraction No. 43) purified with MonoQ and ATP were reacted with each other in a manner similar to Example 7. With the nucleic acid to be added being any of M13 ssDNA, pBluescript, the whole RNA derived from rat liver cell (Total RNA), PolyA, PolyG, PolyU, PolyC, and no addition, the ATPase activity of TIP49 was investigated with the liquid scintillation counter.

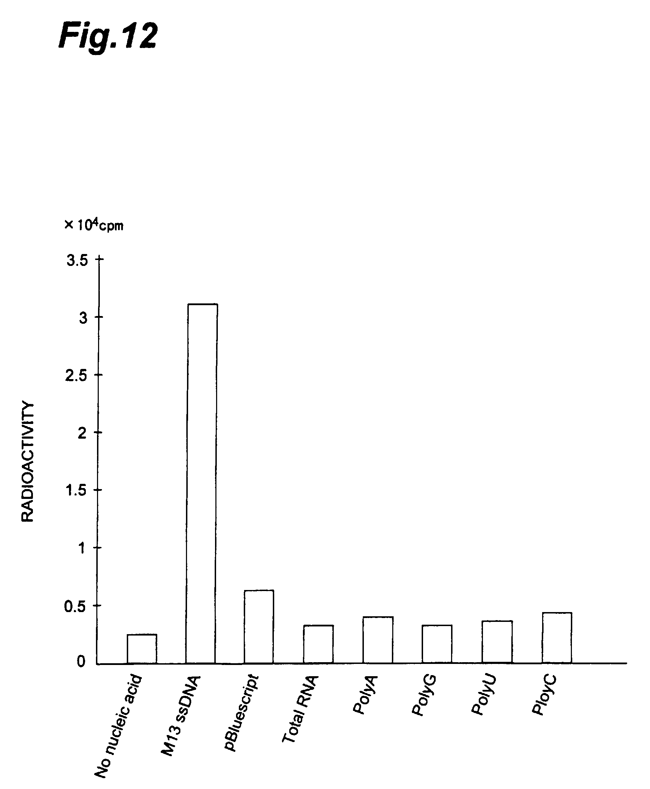

The results are shown in Fig. 12. From Fig. 12, it has been found that the ATPase activity of TIP49 is somewhat activated with the addition of nucleic acid and in particular with M13 ssDNA.

### (Example 9) Confirmation of Helicase Activity of DNA in TIP49

1) The eluate from fractions No. 38 to No. 47 purified with MonoQ and a sample DNA in which a single-strand DNA of 30b (CAGTCACGAC GTTGTAAAAC GACGGCCAGT) had been hybridized with M13 ssDNA were reacted with each other, with addition of ATP, for 30 minutes at 37°C in a reaction liquid having the following composition. For comparison, the eluate purified with MonoQ and the sample DNA were similarly reacted with each other in cases where no ATP was added, ADP was added, and no MgCl₂ was added.
   20 mM Tris-HCl pH 7.5
   2 mM DTT
   50 µg/µl BSA
   0.5 mM MgCl₂
   80 mM KCl
   1 mM ATP
   10 to 15 ng (1500 cpm) substrate (M13 ssDNA)
   enzyme (MonoQ purified eluate)
   SP to yield a total volume of 20 µl
2) To the reaction liquid, 5 µl of a stop liquid having the following composition were added, so as to stop the reaction.
   60 mM EDTA
   0.75% SDS
   0.1% BPB
   50% glycerol
3) The reaction liquid was subjected to electrophoresis on 10% acrylamide/0.5 x TBE (Tris-borate/EDTA) with 120 V of voltage applied thereto.
4) After the completion of electrophoresis, the gel was dried, and autoradiography (with an exposure time of 12 hours) was effected.

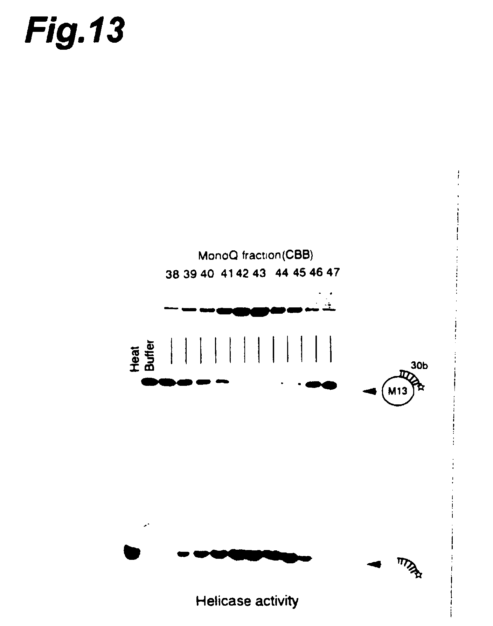

The results are shown in Fig. 13. From Fig. 13, it has been found that, since a band of single-strand DNA of 30b dissociated from the sample DNA is detected in fractions (No. 42 to No. 45) containing a large amount of TIP49, the TIP49 in these fractions dissociates the association between M13 and the single strand DNA of 30b. Namely, such TIP49 has a DNA helicase activity.

Also, it has been found that TIP49 has no DNA helicase activity when ATP is not added and when Mg²⁺ is not added.

### (Example 10) Acquisition of Human TIP49 Gene

### 1. Making of Human cDNA Library

By using a human liver cDNA library (manufactured by Clonetech Laboratories, Inc.), plaques on the order of 10⁶ were made on an NZY agar culture. This cDNA library was fixed onto a nitrocellulose membrane (manufactured by Millipore Corp.) according to the method described in "Molecular Cloning Second Edition" (Cold Spring Harbor Laboratory Press, 1989), Chapter 9. Thereafter, this membrane was washed with 2 x SSC at 60°C.

### 2. Acquisition of Human TIP49 Gene

By using TIP49 DNA (whole length) as a probe, the whole length of human TIP49 gene was obtained from the human cDNA library in a manner similar to 5. of Example 1.

### 3. Introduction of human TIP49 gene into Escherichia coli.

In a manner similar to 8. of Example 1, the human TIP49 gene obtained above was introduced into E. coli JM109. Thereafter, E. coli JM109 was collected.

The recombinant Escherichia coli into which TIP49 gene had been introduced was deposited in National Institute of Bioscience and Human-Technology, Agency of Industrial Science and Technology, Ministry of International Trade and Industry (1-3, Higashi 1-chome, Tsukuba-shi, Ibaragi-ken, postal code 305-8566, Japan) on June 23, 1997 (accession number: FERM P-16281), and then transferred to international deposit on May 25, 1998 (accession number: FERM BP-6376).

### 4. Determination of Base Sequence of Human TIP49 Gene

In a manner of similar to 9. in Example 1, by using Dye Terminator FS Sequence Kit (manufactured by Perkin-Elmer, Inc.) and antosequencer ABI3739, the whole base sequence of human TIP49 gene was determined. The results are shown in SEQ ID NO. 4 in the Sequence Listing.

### 5. Determination of Amino Acid Sequence of Human TIP49

From the base sequence determined in 4., the amino acid sequence of human TIP49 was determined. The results are shown in SEQ ID NO. 3 in the Sequence Listing.

The homology between rat TIP49 gene and human TIP49 gene is 90.3% in the code region of protein. The rat TIP49 protein and the human TIP49 protein differ from each other only in the amino acid at site 291, and are conserved very well between the species. It suggests that TIP49 is an essential material for organisms.

### Industrial Applicability

The TIP49 protein, mutants of the TIP49 protein, polynucleotides coding them, antisense polynucleotides of these polynucleotides, and an antibody recognizing the above-mentioned protein in accordance with the present invention provide a means for elucidating the transcription control function of TBP involved in transcription of all the genes of organisms.

The TIP49 of the present invention can also be used as DNA helicase and ATPase.

The TIP49 of the present invention can also be used for detecting DNA disorders.

## Claims

1. A protein selected from the following groups (a) and (b):
(a) a protein comprising the amino acid sequence set forth in SEQ ID NO.1 in the Sequence Listing;
(b) a protein having the activity of forming a complex with TBP and comprising the amino acid sequence modified by substitution, deletion or addition of one or plural amino acid(s) in the amino acid sequence set forth in SEQ ID NO.1 in the Sequence Listing.

2. A protein selected from the following groups (c) and (d):
(a) a protein comprising the amino acid sequence set forth in SEQ ID NO.3 in the Sequence Listing;
(b) a protein having the activity of forming a complex with TBP and comprising the amino acid sequence modified by substitution, deletion or addition of one or plural amino acid(s) in the amino acid sequence set forth in SEQ ID NO.3 in the Sequence Listing

3. A protein of claim 1 or 2 further having the ATPase activity.

4. A protein of any one of claims 1 to 3 further having DNA helicase activity.

5. A protein selected from the following groups (e) and (f):
(e) a protein having the activity of forming a complex with TBP and comprising a part of the amino acid sequence set forth in SEQ ID NO.1 in the Sequence Listing;
(f) a protein having the activity of forming a complex with TBP and comprising a part of the amino acid sequence modified by substitution, deletion or addition of one or plural amino acid(s) in the amino acid sequence set forth in SEQ ID NO.1 in the Sequence Listing.

6. A protein selected from the following groups (g) and (h):
(g) a protein having the activity of forming a complex with TBP and comprising a part of the amino acid sequence set forth in SEQ ID NO.3 in the Sequence Listing;
(h) a protein having the activity of forming a complex with TBP and comprising a part of the amino acid sequence modified by substitution, deletion or addition of one or plural amino acid(s) in the amino acid sequence set forth in SEQ ID NO.3 in the Sequence Listing.

7. A protein of claim 5 or 6 further having the ATPase activity.

8. A protein of any one of claims 5 to 7 further having DNA helicase activity.

9. DNA coding the protein of any one of claims 1 to 8.

10. DNA comprising the base sequence set forth in SEQ ID NO.2 or 4.

11. RNA coded by the DNA of claim 9 or 10.

12. An antisense DNA corresponding to the DNA of claim 9 or 10, an antisense RNA corresponding to the RNA of claim 11, or an antisense polynucleotide comprising a derivative thereof.

13. A polynucleotide comprising a part of the polynucleotide of any one of claims 9 to 12 and further comprising 12 or more consecutive bases.

14. A polynucleotide that the polynucleotide of any one of claims 9 to 13 as chemically modified.

15. A method obtaining cDNA which is a homologue of the DNA of claim 10, wherein using the polynucleotide of claim 12 or 13 as a probe, cDNA hybridizing to said polynucleotide is obtained from a cDNA library.

16. cDNA which is a homologue of the DNA of claim 10, and which is obtained by the method of claim 15.

17. A protein which is a homologue of the protein of any one of claims 1 to 3, comprising an amino acid sequence coded by the cDNA of claim16.

18. An antibody recognizing the protein of any one of claims 1 to 8 and 17.
